# FASCICULE DE BREVET EUROPEEN

(11) **EP 0 418 335 B1**
(45) Date de publication et mention de la délivrance du brevet: **28.05.1997**
(21) Numéro de dépôt: 90902287.3
(22) Date de dépôt: 26.01.1990
(51) Int. Cl.: G01N 33/50, C12Q 1/18

(54) **PROCEDE D'EVALUATION DE L'ACTIVITE ANTI-OXYDANTE D'UN AGENT CHIMIQUE OU PHYSIQUE AU MOYEN DE RADICAUX LIBRES**
VERFAHREN ZUR BESTIMMUNG DER ANTIOXIDATIVEN AKTIVITÄT EINES CHEMISCHEN ODER PHYSIKALISCHEN AGENS MITTELS FREIER RADIKALE
PROCESS FOR THE EVALUATION OF THE ANTI-OXIDANT ACTIVITY OF A CHEMICAL OR PHYSICAL AGENT BY MEANS OF FREE RADICALS

(30) Priorité: 27.01.1989 FR 8900999
(43) Date de publication de la demande: 27.03.1991
(73) Titulaire: SPIRAL RECHERCHE ET DEVELOPPEMENT, F-21560 Dijon Cédex (FR)
(72) Inventeur: PROST, Michel, F-21560 Couternon (FR)
(74) Mandataire: Clisci, Serge
(86) Numéro de dépôt international: FR9000061
(87) Numéro de publication internationale: WO9008955

(56) Documents cités:
- CHEMICAL ABSTRACTS, Vol. 107, No. 23, 7 December 1987, (Columbus, Ohio, US), M. MIKI et al.: "Free-Radical Chain Oxidation of Rat Red Blood Cells by MolecuLar Oxygen and its Inhibition by alpha-Tocopherol", voir pages 186* Resume 213235v, & Arch. Biochem. Biophys. 1987, 258(2), 373-80*
- CHEMICAL ABSTRACTS, Vol. 107, No. 25, 21 December 1987, ( Columbus, Ohio, US), V.N. USHKALOVA et al.: "Spectrophotometry, Fluorometry, and Kinetic Methods used for Analysis of Blood Lipid Free Radicals", voir pages 415* Resume 232454g, & Lab. Delo 1987, (6), 446-50*
- CHEMICAL ABSTRACTS, Vol. 100, No. 13, 26 Mars 1984, (Columbus, Ohio, US), E.B. SPEKTOR et al.: "Determination of the Total Antioxidizing Activity of the Blood Plasma and Spinal Fluid", voir pages 311-312* Resume 99345J, & Lab. Delo 1984, (1), 26-8*
- Biological Abstracts, Vol. 72, No. 9, 1981, (Philadelphia, PA., US), R.A. LOVSTAD: "The Protective Action of Ceruloplasmin on Iron (II) Stimulated Lysis of Rat Erythrocytes", voir pages 5914, Resume 57169, & Int. J. Biochem 13(2): 221-224. 1981
- Biological Abstracts, Vol. 73, No. 12, 1982, (Philadelphia, PA., US), B.N. AMES et al.: "Uric Acid Provides an Antioxidant Defense in Humans against Oxidant- Caused and Radical-Caused Aging and Cancer: a Hypothesis", voir page 8817, Resume 84420, & Proc. Natl. Acad. Sci. U.S.A. 78(11): 6858-6862. 1981

## Description

### DOMAINE DE L'INVENTION

La présente invention a trait à un nouveau procédé mettant en oeuvre la lyse d'un matériau cellulaire au moyen de radicaux libres.

Elle concerne plus précisément le procédé d'évaluation de l'activité anti-oxydante d'un agent chimique ou physique, susceptible soit d'augmenter ou accélérer, soit d'inhiber ou retarder la lyse cellulaire induite par des radicaux libres.

### ART ANTERIEUR

On sait que les radicaux libres ont en général un effet néfaste sur l'organisme et en particulier au niveau des cellules de cet organisme. Les radicaux libres attaquent la paroi cellulaire plus ou moins rapidement en fonction de la résistance des cellules que confère le matériel (ou équipement) enzymatique et moléculaire desdites cellules. Lorsque la paroi cellulaire a été dégradée, perforée ou ouverte par des radicaux libres, le contenu de la cellule se répand à l'extérieur de la paroi. Voir en particulier les documents suivants : Chemical Abstracts 107, 213235v, Chemical Abstracts 107, 232454g, Chemical Abstracts 100, 99345j, Biological Abstracts 72 (No. 9), page 5914 résumé No. 57169, (1981) et Biological Abstracts 73 (No. 12), page 8817, résumé No. 84420, (1982).

Le résumé CA 107, 213235v, qui se réfère à un article de M. MIKI et al., Arch. Biochem. Biophys., 258 (No. 2), pages 373-380 (1987), signale que l'alphatocophérol protège les hématies de rat vis-à-vis de la lyse induite par les radicaux libres.

Selon le résumé CA 100, 99345j précité, qui se réfère à un article de E.B. SPEKTOR et al., Lab. Delo (No. 1), pages 26-28 (1984), l'évaluation de l'activité anti-oxydante totale d'un échantillon (plasma sanguin ou liquide spinal) est déterminée par absorption à 532 nm après induction de radicaux libres au niveau d'un matériau cellulaire (dans le cas d'espèce : des membranes d'érythrocytes) au moyen d'une lampe UV.

Selon l'invention, on préconise une nouvelle solution technique selon laquelle (i) les radicaux libres ne sont pas générés au niveau dudit matériau cellulaire, mais proviennent d'un initiateur de radicaux libres ajouté audit matériau cellulaire, et (ii) le matériau cellulaire a été préalablement mis en contact avec un agent chimique ou physique.

### OBJET DE L'INVENTION

Selon l'invention, on préconise un nouveau procédé d'évaluation de l'activité anti-oxydante d'un agent chimique ou physique à tester au moyen d'un matériau cellulaire d'activité anti-oxydante connue, ledit procédé, qui comprend l'utilisation de radicaux libres oxydants en tant que moyen induisant la lyse cellulaire, étant caractérisé en ce que
1°) on met en contact dans un milieu biologique liquide approprié, un générateur de radicaux libres et un matériau cellulaire (I) choisi parmi l'ensemble constitué par
   (a) les cellules humaines, animales et végétales,
   (b) les fragments desdites cellules, et
   (c) les parois synthétiques contenant des liposomes,
   ledit matériau cellulaire, qui comporte un colorant libérable lors de la lyse par les radicaux libres, ayant été préalablement mis en contact avec un agent chimique ou physique (II) ;
2°) on induit la libération des radicaux libres à partir dudit générateur de radicaux libres ; et,
3°) on évalue la lyse du matériau cellulaire par une mesure de densité optique, par rapport à un échantillon témoin contenant ledit matériau cellulaire qui n'a pas été mis en contact avec ledit agent chimique ou physique.

Selon ce procédé, on observe la lyse ou l'état oxydatif du matériau cellulaire I pour apprécier l'influence de l'agent II sur la lyse dudit matériau I.

La lyse du matériau cellulaire peut être notamment suivie de façon "cinétique" [par mesure à des temps réguliers sur des prélèvements (d'un volume constant) de milieu liquide d'essai contenant le générateur de radicaux libres, le matériau cellulaire et l'agent II à tester] ou de façon "non-cinétique" [par mesure du type dose-réponse sur des échantillons du milieu liquide d'essai contenant le matériau cellulaire, associé à l'agent II à tester, et des quantités aliquotes croissantes du générateur de radicaux libres].

Dans le cadre de l'évaluation cinétique, la résistance anti-radicaux libres du matériau cellulaire I est exprimée par le temps correspondant à une lyse de 50 % dudit matériau cellulaire.

Dans le cadre de l'évaluation dose-réponse, la résistance anti-radicaux libres du matériau cellulaire est exprimée par la concentration de générateur de radicaux libres induisant une lyse de 50 % dudit matériau cellulaire.

### DESCRIPTION DETAILLEE DE L'INVENTION

Parmi les générateurs de radicaux libres qui conviennent selon l'invention, on peut notamment signaler les produits qui libèrent des radicaux libres et qui sont utilisés couramment dans le domaine de la polymérisation pour l'obtention de macromolécules. Parmi ces produits on peut notamment citer les générateurs de radicaux libres oxydants, tels que le peroxyde de benzoyle, les perbenzoates d'alkyle en C₁-C₈, de préférence en C₃-C₄ (notamment les perbenzoates de n-butyle, t-butyle, i-propyle et n-propyle), les peroxydicarbonates de dialkyle où les groupes alkyle contiennent de 1 à 8 atomes de carbone, de préférence de 3 à 4 atomes de carbone (notamment le peroxydicarbonate de diisopropyle), l'hydroxyperoxyde de cumène, l'azo-bis(isobutyronitrile), le 2,2'-azo-bis (2,4-diméthylvaléronitrile), le 2,2'-azo-bis(2-amidinopropane) et leurs éventuels sels d'addition tels que les chlorhydrates, et, leurs analogues.

Parmi les générateurs de radicaux libres oxydants on préfère ceux qui présentent une cinétique ou vitesse de réaction d'ordre O (la libération des radicaux libres est constante dans le temps) et mieux d'ordre 1 (la libération des radicaux libres est linéaire dans le temps). Les générateurs de radicaux libres oxydants préférés selon l'invention sont, d'une part, le dichlorhydrate de 2,2'-azo-bis(2-amidinopropane) qui donne dans un milieu aqueux une cinétique d'ordre 1, et d'autre part, le 2,2'-azo-bis(2,4-dimethylvaléronitrile) qui donne dans un Milieu liquide huileux ou organique une cinétique d'ordre 1. La libération des radicaux libres à partir d'un générateur de radicaux libres est réalisée selon une méthode connue en soi, par exemple la chaleur, la lumière (notamment la lumière du spectre visible ou les UV), les protons, les électrons, les rayons X; de préférence cette libération sera initiée par des photons, ou la chaleur. Par exemple, le fait de porter le dichlorhydrate de 2,2'-azo-bis(2-amidinopropane) en solution aqueuse à la température de 37°C suffit pour déclencher la libération de radicaux libres oxydants selon la réaction

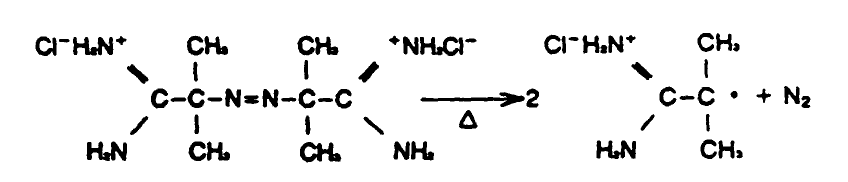

Le matériau cellulaire I selon l'invention comprend des cellules d'origine humaine, animale, végétale ou synthétique, ou des fragments desdites cellules tels que les parois.

De façon avantageuse, on utilisera un matériau I contenant un marqueur coloré ou fluorescent libérable sous l'action des radicaux libres.

Parmi les matériaux cellulaires I qui conviennent selon l'invention on fera notamment appel à des cellules d'origine humaine, animale ou végétale pigmentées, c'est-à-dire contenant un pigment ou colorant tel que l'hémoglobine, la chlorophylle, la xanthophylle, le carotène et les anthocyanes dont la libération lors de la lyse cellulaire est détectable notamment par la mesure de la variation de la densité optique au moyen d'un spectrophotomètre. Les cellules que l'on recommande sont des hématies d'origine animale et de préférence des hématies prélevées sur des animaux à sang chaud tels que les mammifères, notamment l'homme. Le choix des hématies repose sur les éléments suivants :
- les cellules sanguines telles que les hématies ont une demi-vie relativement courte (80 jours pour les hématies d'origine humaine);
- les hématies possèdent tout l'équipement moléculaire et enzymatique de la protection anti-radicalaire et, de ce fait, elles sont considérées comme représentatives des autres cellules de l'organisme ; et,
- l'accessibilité et la grande disponibilité des hématies, notamment par le biais d'une simple prise de sang d'un volume de quelques millilitres.

Quand on soumet des hématies isolées de leur plasma à une agression de type oxydatif avec des radicaux libres, les hématies mettent en jeu tout leur équipement enzymatique et moléculaire pour résister à cette agression jusqu'à ce que la membrane ou paroi cellulaire en soit modifiée au point de laisser échapper le contenu cellulaire. Dans le cas des hématies, cette libération dudit contenu peut être facilement déterminée par spectrophotométrie en mesurant la quantité d'hémoglobine passant dans le milieu biologique. La résistance de la population d'hématies que l'on teste s'exprime soit par le temps de libération de 50 % p/p de l'hémoglobine contenue dans les hématies (évaluation cinétique), soit par la concentration de générateur de radicaux libres (CH_{50 %}) engendrant 50 % d'hémolyse (évaluation dose-réponse).

Le matériau cellulaire peut également être constitué par des parois ou des fragments de parois de cellules de préférence pigmentées. Les parois qui conviennent le mieux sont celles qui contiennent une quantité suffisante de pigment ou colorant libérable lors de la lyse par les radicaux libres.

Le matériau cellulaire peut également être constitué par une paroi cellulaire synthétique. Parmi les parois synthétiques, on préfère les substances contenant des liposomes dans lesquels est enrobé, fixé ou immobilisé un moyen de marquage coloré libérable lors de la lyse par les radicaux libres. Les substances membranaires contenant des liposomes et de tels marqueurs colorés sont particulièrement avantageuses en ce sens qu'elles permettent d'éviter de rechercher des sujets humain, animal ou végétal sains pour les essais de contrôle, et assurent ainsi une meilleure standardisation du procédé de dosage selon l'invention.

De façon préférée selon l'invention, le matériau cellulaire à utiliser sera constitué par des hématies, et mieux par des liposomes masquant, enrobant, fixant ou immobilisant un marqueur coloré libérable au cours de la lyse par les radicaux libres. La résistance anti-radicaux libres que l'on teste s'exprimé alors soit par le temps de lyse à 50 % c'est-à-dire le temps de libération de 50 % p/p du marqueur coloré (mesure cinétique), soit par la concentration de générateur de radicaux libres induisant une lyse à 50 % (mesure dose-réponse).

Par matériau cellulaire "contaminé" par un agent II, on entend un matériau cellulaire I, tel que défini ci-dessus, qui a été mis en contact avec ledit agent II au moins 0,5 h avant la mise en oeuvre du procédé de l'invention.

L'agent II peut être de nature physique (irradiation avec des rayons X, des rayons béta, des protons, etc...) ou chimique (substances à tester ou substances de référence, métabolites, etc...), voire encore physico-chimique (fumée de tabac impliquant notamment l'émission de radicaux libres par pyrolyse).

Le milieu biologique d'essai, qui est un milieu liquide aqueux ou un milieu liquide organique, comprend le matériau cellulaire I préalablement "contaminé" par l'agent II, un générateur de radicaux libres, et, le cas échéant, un ou plusieurs additifs usuels dans le domaine des cultures cellulaires et des dosages biologiques, notamment un moyen conservateur.

Lorsque le moyen II est une substance chimique, cette dernière peut être une substance à tester quelconque. Il peut s'agir notamment d'une substance oxydante, d'une substance anti-oxydante, d'une composition ou association de produits ou encore d'un métabolite. Parmi les substances que l'on peut tester et/ou doser, on peut également signaler les pigments (notamment les flavonoïdes), les protéines, les enzymes, les peptides, les aminoacides, les anticorps et les antigènes, et d'une manière générale, tous produits à partir desquels on peut générer des anticorps. Parmi ces substances susceptibles d'être testées et/ou dosées, on peut en particulier citer les produits ou moyens intervenant sur l'organisme et/ou les cellules.

Pour la mise en oeuvre du procédé selon l'invention, on met en suspension le matériau cellulaire isolé et lavé dans un milieu biologique liquide de préférence isotonique. On incube à une température située dans la gamme allmt de 10 à 60°C, de préférence de 15 à 40°C et mieux à une température de 37°C, en présence d'un générateur de radicaux libres, à raison de 50 à 200 mM de générateur de radicaux libres pour une concentration de 10 à 20 % p/v de matériau cellulaire.

Le meilleur mode de mise en oeuvre du procédé de l'invention consiste à mettre en suspension, soit un matériau cellulaire constitué par des hématies, soit des fragments de paroi synthétique contenant des liposomes et un marqueur coloré libérable, dans un sérum physiologique isotonique le cas échéant tamponné, et à incuber à 37°C en présence de dichlorhydrate de 2,2'-azo-bis(2-amidinopropane) à la concentration de 100 mM pour un volume final de 2 ml de milieu biologique liquide aqueux (de préférence) ou de 2,2'-azo-bis(2,4-diméthylvaléronitrile) dans les mêmes proportions pour un milieu liquide biologique organique. La libération des radicaux libres à partir du générateur de radicaux libres est initiée de préférence par des photons et mieux par un choc thermique (dans le cas d'espèce un chauffage à 37°C). Des prélèvements (0,02 ml) sont effectués à intervalles de temps réguliers (par exemple toutes les 20 minutes) jusqu'à ce que le culot cellulaire ait disparu (soit une durée de l'ordre de 150 à 600 minutes en général, et notamment de 200-300 minutes); chaque prélèvement est dilué au moyen de 1 ml de sérum physiologique et centrifugé [par exemple 10-60 secondes à 3000-9000 g, de préférence à 3000-6000 g (une centrifugation trop poussée, notamment supérieure à 9000 g peut perturber la mesure en induisant une lyse mécanique)]; une partie aliquote (0,2 ml) du surnageant ainsi obtenu est alors transférée dans les puits d'une microplaque ou d'un ensemble de microcuvettes pour lecture de la densité optique par spectrophotométrie (notamment à 350-600 nm, quelle que soit l'origine des cellules pigmentées ou des parois synthétiques, et, en particulier à 405-410 et 540 nm, lorsque le matériau cellulaire est constitué par des hématies).

En pratique les résultats des variations de la densité optique sont exprimés en pourcentage par rapport à la lyse maximale du matériau cellulaire (100 %). Une courbe théorique ajustée aux points expérimentaux permet d'obtenir en particulier le temps correspondant à 50 % de lyse (ce temps est d'autant plus long que les hématies ou les parois synthétiques résistent mieux au stress oxydatif in vitro conduit dans les conditions opératoires précitées), la pente de la sigmoïde du pic et le temps de latence (déterminé par la tangente du point d'inflexion de ladite sigmoïde).

L'évaluation cinétique décrite ci-dessus peut être remplacée par une évaluation du type dose-réponse.

Le procédé selon l'invention est de mise en oeuvre très simple et peut être commercialisé sous forme de nécessaire, trousse ou kit de dosage soit à des fins diagnostiques, soit pour des études de "screening" de diverses molécules et de leurs métabolites, notamment des molécules ayant une activité oxydante ou anti-oxydante, d'une part, et des molécules ayant une action à long terme, d'autre part. Le procédé peut également être effectué sur du plasma humain ou animal de façon à apprécier l'influence desdites molécules sur ledit plasma constituant alors dans un tel dosage ledit agent Il.

Le procédé de l'invention est particulièrement intéressant pour (i) le dosage des agents anti-malaria qui sont d'une manière générale des substances réduisant la résistance des hématies et des liposomes aux radicaux libres, (ii) le diagnostic du diabète dès lors que cette maladie produit dans l'organisme un effet hypo-oxydatif, et (iii) apprécier l'influence d'aliments ou d'additifs alimentaires, notamment les colorants, sur l'organisme.

Ce procédé présente l'avantage d'étre mis en oeuvre avec des durées très courtes, de préférence inférieures ou égales à 5 h.

Pour l'utilisation de liposomes en tant que matériel cellulaire selon l'invention on préconise les préparations liposomiques suivantes.

### PREPARATION A :

Particules renfermant en association des liposomes, un agent coloré ou fluorescent de marquage libérable sous l'action des radicaux libres, et un moyen liant assurant la cohésion de chaque particule.

### PREPARATION B :

Matrice liposomique constituée par une association de liposomes, d'un agent coloré ou fluorescent de marquage libérable sous l'action des radicaux libres, et un moyen liant assurant la cohésion de la matrice.

### PREPARATION C :

Support inerte sur lequel est adhésivement liée une couche liposomique constituée de la matrice selon la préparation B ci-dessus.

### PREPARATION D :

Support inerte comportant, sur au moins une de ses faces, au moins une zone revêtue d'un agent coloré ou fluorescent de marquage libérable sous l'action des radicaux libres, ladite face et ladite zone étant revêtues par une couche constituée d'un mélange de liposomes et d'agent liant assurant la cohésion de ladite couche ; ici la couche liposomique masque la zone revêtue du marqueur coloré ou fluorescent.

### PREPARATION E :

Support inerte poreux renfermant un agent coloré ou fluorescent de marquage libérable sous l'action des radicaux libres qui a été apporté par imprégnation, ledit support étant revêtu d'une couche liposomique analogue à celle de la préparation D masquant ledit marqueur.

### PREPARATION F :

Matrice liposomique selon la préparation D sensibilisée en surface par un marqueur chromogène d'un type connu dans le domaine des substrats peptidiques chromogènes (voir notamment EP-A-0 280 610); sous l'action des radicaux libres des fragments ou débris de liposomes comportant ledit marqueur sont séparés de la matrice, on isole ces fragments ou débris de la matrice et on les recueille par filtration ou centrifugation, puis lesdits fragments ou débris sont remis en suspension dans un milieu biologique approprié pour développer la coloration selon une technique connue (voir EP-A-0 280 610 précité).

Dans les préparations D-E ci-dessus, l'agent coloré ou fluorescent de masquage libérable sous l'action des radicaux libres est physiquement masqué par la matrice liposomique dont l'épaisseur doit être relativement faible pour avoir aisément accès audit marqueur sous l'action des radicaux libres. Dans les préparations A-C on peut avoir, soit un masquage purement physique de l'agent coloré ou fluorescent de marquage libérable sous l'action des radicaux libres, soit une association dudit marqueur avec les liposomes plus élaborée telle que fixation, immobilisation ou séquestration.

D'autres avantages et caractéristiques de l'invention seront mieux compris à la lecture qui va suivre d'exemples de réalisation. L'ensemble de ces éléments n'est nullement limitatif mais est donné à titre d'illustration. Les exemples 1-9 concernent des dosages cinétiques et les exemples 10-15 des dosages du type dose-réponse.

### EXEMPLE 1

Pour quantifier le procédé selon l'invention on a étudié l'influence de la concentration du dichlorhydrate de 2,2'-azo-bis(2-amidinopropane) sur des hématies de rat.

Après prélèvement et lavage, les hématies de rat sont mises en suspension (hématocrite 15 % p/v) dans du sérum physiologique isotonique. Ces hématies sont mises en contact avec le dichlorhydrate de 2,2'-azo-bis(2-amidinopropane) à des doses de 0 mM (contrôle), 25 mM, 50 mM et 100 mM, le volume final du milieu biologique liquide aqueux étant de 2 ml. On initie la libération des radicaux libres par la chaleur en portant le milieu réactionnel à 37°C. On effectue des prélèvements de 0,02 ml toutes les 20 minutes pendant 200-240 minutes, chaque prélèvement étant dilué dans 1 ml de sérum physiologique et centrifugé (15 secondes; 4000 g). Une partie aliquote (0,2 ml) du surnageant est alors transférée dans les puits d'une microplaque pour lecture de la densité optique (notamment à 540 mn) par spectrophotométrie.

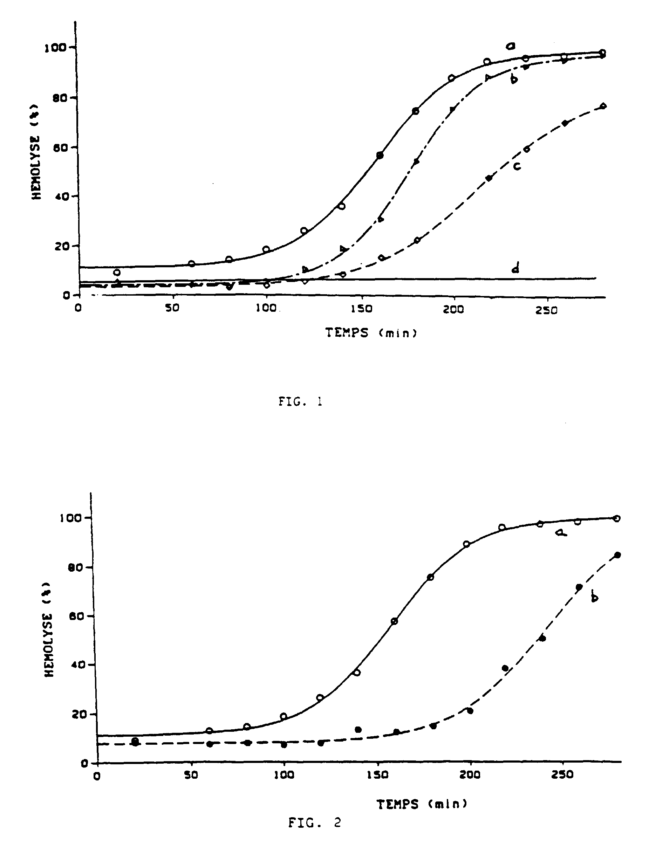

Les résultats consignés dans la figure 1, sont exprimés par le temps (en minutes), correspondant à un taux de 50 % d'hémolyse (t_{50 %}). La courbe (a) est relative à la concentration de 100 mM de dichlorhydrate de 2,2'-azo-bis(2-amidinopropane) et donne une valeur t_{50 %} = 158 minutes; la courbe (b) est relative à la concentration de 50 mM de dichlorhydrate de 2,2'-azo-bis(2-amidinopropane) et donne une valeur t_{50 %} = 176 minutes ; la courbe (c) est relative à la concentration de 25 mM de dichlorhydrate de 2,2'-azo-bis(2-amidinopropane) et donne une valeur t_{50 %} = 214 minutes; et la courbe (d) est relative à l'essai contrôle, c'est-à-dire à l'absence de dichlorhydrate de 2,2'-azo-bis(2-amidinopropane).

Les résultats de la figure 1 mettent en évidence la relation dose/dépendance de l'effet sur la lyse cellulaire par rapport à la molécule génératrice de radicaux libres.

### EXEMPLE 2

Le présent exemple concerne l'effet d'un agent anti-oxydant, dans le cas d'espèce l'acide ascorbique.

On procède selon les modalités opératoires décrites à l'exmple 1 ci-dessus en utilisant un milieu biologique liquide aqueux (sérum physiologique) contenant des hématies de rat, 100 mM de dichlorhydrate de 2,2'-azo-bis(2-amidinopropane) et 0 (absence d'agent antioxydant) ou 0,1 mM d'acide ascorbique, la libération des radicaux libres étant initiée 0,5 h après mise en contact de l'acide ascorbique avec les hématies et le générateur de radicaux libres.

Les résultats consignés dans la figure 2 montrent que, en l'absence d'acide ascorbique [courbe (a)], la valeur t_{50 %} est égale à 158 minutes, et que en présence de 0,1 mM d'acide ascorbique [courbe (b)], la valeur t_{50 %} est égale à 244 minutes. En d'autres termes, la présence d'un antioxydant tel que l'acide ascorbique retarde la lyse cellulaire provoquée par les radicaux libres.

### EXEMPLE 3

Le présent exemple concerne l'effet d'un agent anti-oxydant incorporé au niveau de la membrane d'hématies de rat, à savoir le butylhydroxytoluène [en abrégé : HBT; nomenclature systématique : 2,6-di-(1,1-diméthyléthyl)-4-méthylphénol].

On procède suivant les modalités opératoires décrites à l'exemple 1, en utilisant des hématies de rat sain (contrôle) et des hématies préalablement préincubées à 37°C pendant 0,5 h en présence de BHT et de son solvant, l'éthanol (pour adsorption du BHT au niveau de la membrane cellulaire des hématies) puis remises en suspension pour effectuer le dosage. Les hématies ainsi traitées sont placées dans le sérum physiologique de l'exemple 1 et mises en contact avec 100 mM de dichlorhydrate de 2,2'-azo-bis (2-amidinopropane), le milieu réactionnel résultant étant ensuite porté à 37°C.

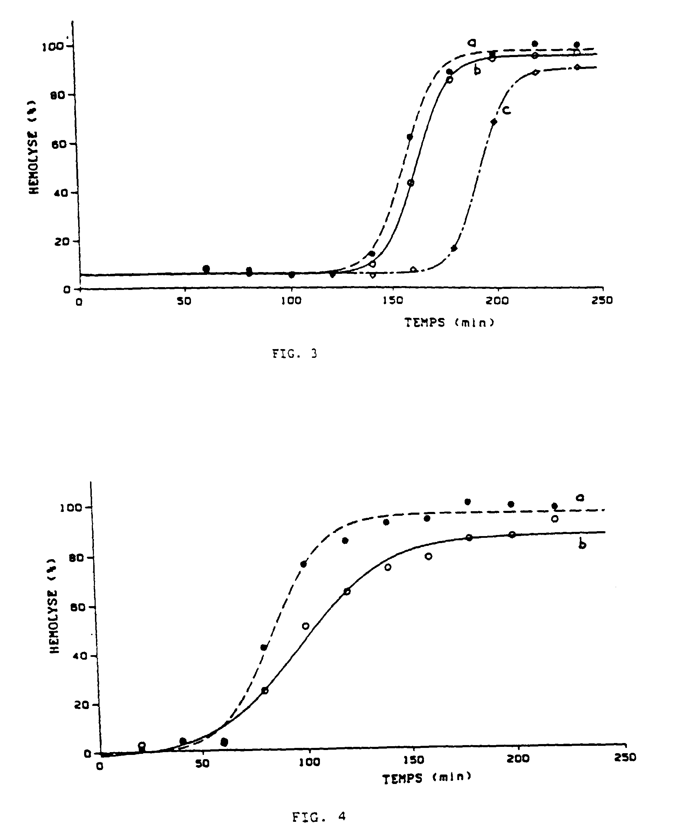

Les résultats sont consignés dans la figure 3. La courbe contrôle (a) donne un t_{50 %} égal à 163 minutes. La courbe (b) relative aux hématies de rat sain préincubées avec le solvant du BHT, à savoir de l'éthanol à la concentration de 0,5 % p/v, pendant 0,5 h à 37°C, donne une valeur t_{50 %} égale à 157 minutes. La courbe (c) relative aux mêmes hématies que la courbe (b) mais préincubées dans les mêmes conditions avec le BHT et de l'éthanol (le milieu d'essai contenant 0,03 mM de BHT et 0,5 % p/v d'éthanol) donne une valeur en t_{50 %} égale à 193 minutes. La comparaison de la courbe (c) avec les courbes (a) et (b) démontre la protection que confère le BHT vis-à-vis de la lyse cellulaire. En d'autres termes, l'agent antioxydant utilisé ici retarde dans le temps la lyse cellulaire provoquée par les radicaux libres.

### EXEMPLE 4

Le présent exemple a trait à la comparaison d'hématies provenant de deux sujets différents. Ces hématies, après séparation et lavage, sont remises en suspension à la même concentration pour être soumises à l'action de radicaux libres provenant d'un générateur de radicaux libres, dans le cas d'espèce 100 mM de dichlorhydrate de 2,2'-azo-bis(2-amidinopropane).

Les résultats sont consignés dans la figure 4. La courbe (a) représente le pourcentage d'hémolyse d'hématies d'un fumeur adulte et donne une valeur t_{50 %} égale à 84,4 minutes. La courbe (b) représente le pourcentage d'hémolyse d'un non-fumeur adulte et donne une valeur t_{50 %} égale à 93,3 minutes. La comparaison des courbes (a) et (b) montre que chez le fumeur les résistances normales vis-à-vis de l'agression par des radicaux libres sont diminuées par rapport aux sujets non-fumeurs.

### EXEMPLE 5

Le présent exemple a trait à l'utilisation de cellules végétales pour apprécier l'influence d'une irradiation sur l'état oxydatif.

Des cellules de camomille sont réparties en deux lots, l'un des deux lots est soumis à une irradiation avec une source radio-active de 3,7 x 10⁶ Bq (100 microcuries), puis le lot irradié et le lot non-irradié sont conservés sous atmosphère inerte (azote ou argon) pendant 7 mois. Les cellules des deux lots sont ensuite mises en suspension dans un milieu biologique liquide aqueux puis mises en contact avec un générateur de radicaux libres, le dichlorhydrate de 2,2'-azo-bis(2-amidinopropane) à 15°C pendant 0,5 h. La libération des radicaux libres est initiée par chauffage à 37-40°C, puis on procède aux prélèvements et analyses selon les modalités de l'exemple 1. L'étude cinétique de la lyse du matériau cellulaire montre que les cellules préalablement irradiées ont une résistance aux radicaux libres inférieure à celle des cellules non-irradiées. Les courbes correspondantes sont similaires à celles de la figure 3 (la cinétique de la lyse des cellules irradiées et respectivement celle de la lyse des cellules non-irradiées ayant approximativement l'allure des courbes a et respectivement b ou c de ladite figure 3).

### EXEMPLE 6

On irradie un lot de graines de camomille avec une source radio-active de 3,7 x 10⁶ Bq et on conserve ce lot ainsi qu'un lot non-irradié pendant 8 mois sous atmosphère inerte. On broie ensuite les graines irradiées et non-irradiées et les broyats résultants sont mis en contact pendant 1 h à 15°C avec des hématies de rat en suspension dans un sérum physiologique. On introduit ensuite le générateur de radicaux libres, le dichlorhydrate de 2,2'-azo-bis(2-amidinopropane) et procède comme indiqué à l'exemple 1. L'étude de la cinétique de la lyse des hématies en présence de broyat de graines de camomille irradiées, et respectivement non-irradiées, donne des courbes approximativement similaires aux courbes a et respectivement b ou c de la figure 3. Ceci met en évidence que le matériel moléculaire enzymatique des graines a été modifié et dans le cas d'espèce partiellement détruit par l'irradiation.

Le présent exemple 6 et l'exemple 5 ci-dessus illustrent que l'on peut apprécier selon le procédé de l'invention la qualité d'aliments d'origine animale et/ou végétale pour déterminer si oui ou non l'aliment à tester a subi une dégradation plus ou moins importante avant sa consommation.

### EXEMPLE 7

On procède comme indiqué à l'exemple 3 en remplaçant le BHT par la phénothiazine. On constate que la phénothiazine présente un effet anti-oxydant inattendu en ce sens qu'elle retarde la lyse des hématies induite par les radicaux libres.

### EXEMPLE 8

On procède comme indiqué à l'exemple 7 en remplaçant les hématies par un matériau cellulaire synthétique comprenant des liposomes selon la préparation C ci-dessus. On observe comme à l'exemple 7 l'effet anti-oxydant de la phénothiazine.

### EXEMPLE 9

On prépare un lyophilisat de thé par extraction des feuilles séchées avec de l'eau préalablement portée à l'ébullition, et lyophilisation du filtrat résultant, un lot étant soumis à une irradiation avec un rayonnement ionisant (50 kGy) avant lyophilisation, le lot témoin n'est pas irradié. Chaque lot est ensuite conservé sous vide pendant 9 mois.

On procède ensuite comme indiqué à l'exemple 3 en remplaçant le BHT par le lot de thé irradié ou le lot témoin non irradié. On constate que le lot irradié présente une résistance aux radicaux libres inférieure à celle du lot témoin.

### EXEMPLES 10-15

Les exemples 10-15 qui suivent illustrent la mesure de l'activité antiradicalaire dans des conditions de dosage du type dose-réponse

Des quantités aliquotes croissantes (20 à 300 mM) de générateur de radicaux libres [dichlorhydrate de 2,2'-azo-bis(2-amidinopropane)] en solution dans un milieu aqueux (eau ou sérum physiologique) sont placées dans des tubes puis lyophilisées. Après resolubilisation desdites quantités de générateur de radicaux libres dans un volume constant de sérum physiologique d'essai contenant le cas échéant une substance à étudier, on ajoute en quantité constante le matériau cellulaire selon l'invention.

Les milieux d'essai correspondants sont incubés à 37°C pendant une durée de temps définie (2,5 h). L'effet de radicaux libres est ensuite apprécié par la lyse du matériau cellulaire.

Dans l'exemple 10 on a opéré avec des hématies de rat sain, et constaté que la concentration en générateur de radicaux libres (CH_{50 %}) induisant la lyse de 50 % des hématies était de 108,0 + 20 mM/l.

Dans l'exemple 11, on a opéré avec les mêmes hématies de rat sain utilisées à l'exemple 10, le milieu d'essai contenant en outre 10 mM de mannitol. On constate que la CH_{50 %} est égal à 155,6 + 6,1 mM/l ce qui confirme l'effet antioxydant du mannitol par rapport à la CH_{50 %} de l'exemple 10.

Dans l'exemple 12, on a opéré avec les mêmes hématies de rat sain utilisées à l'exemple 10, le milieu d'essai contenant en outre un moyen oxydant, le bis(diméthylamide) de l'acide azodicarboxylique [il s'agit d'un composé oxydant les thiols] à la dose de 250 mM. On constate que ledit moyen peroxydant rend les hématies plus sensibles à l'effet des radicaux libres, pour déplétir du glutathion érythrocytaire, la CH_{50 %} étant abaissée (par rapport à celle de l'exemple 10) à la valeur de 60,8 + 8,1mM/l.

Dans les exemples 13-15 on a obtenu des résultats similaires à ceux des exemples 10-12 en remplaçant les hématies par un matériau cellulaire synthétique contenant des liposomes selon la préparation C ci-dessus.

## Revendications

1. Procédé d'évaluation de l'activité anti-oxydante d'un agent chimique ou physique à tester au moyen d'un matériau cellulaire d'activité anti-oxydante connue, ledit procédé, qui comprend l'utilisation de radicaux libres oxydants en tant que moyen induisant la lyse cellulaire, étant caractérisé en ce que
1°) on met en contact dans un milieu biologique liquide approprié, un générateur de radicaux libres et un matériau cellulaire choisi parmi l'ensemble constitué par
(a) les cellules humaines, animales et végétales,
(b) les fragments desdites cellules, et
(c) les parois synthétiques contenant des liposomes,
ledit matériau cellulaire, qui comporte un colorant libérable lors de la lyse par les radicaux libres, ayant été préalablement mis en contact avec un agent chimique ou physique ;
2°) on induit la libération des radicaux libres à partir dudit générateur de radicaux libres ; et,
3°) on évalue la lyse du matériau cellulaire par une mesure de densité optique, par rapport à un échantillon témoin contenant ledit matériau cellulaire qui n'a pas été mis en contact avec ledit agent chimique ou physique.

2. Procédé suivant la revendication 1, caractérisé en ce que le matériau cellulaire est choisi parmi l'ensemble constitué par les cellules pigmentées d'origine humaine, animale ou végétale.

3. Procédé suivant la revendication 2, caractérisé en ce que lesdites cellules pigmentées sont des hématies d'origine humaine ou animale.

4. Procédé suivant la revendication 1, caractérisé en ce que ledit générateur de radicaux libres est choisi parmi l'ensemble constitué par les produits libérant des radicaux libres selon une cinétique d'ordre 0 ou 1.

5. Procédé suivant l'une quelconque des revendications 1 et 4, caractérisé en ce que ledit générateur de radicaux libres est le dichlorhydrate de 2,2'-azo-bis-(2-amidinopropane).

6. Procédé suivant la revendication 1, caractérisé en ce que l'on met en contact 50 à 200 mM de générateur de radicaux libres avec un matériau cellulaire à une concentration de 10 à 20 % p/v dans un milieu biologique aqueux.

7. Procédé suivant l'une quelconque des revendications 1 et 6, caractérisé en ce que les radicaux libres sont générés par incubation à 37°C.

## Patentansprüche

1. Verfahren zur Bestimmung der antioxidativen Aktivität eines mittels eines Zellmaterials mit bekannter antioxidativer Wirkung zu untersuchenden chemischen oder physikalischen Agens, welches den Einsatz freier Radikale als Mittel umfaßt, welches die Zellenlyse bewirkt, **dadurch gekennzeichnet,** daß
1) ein Erzeuger freier Radikale sowie ein Zellmaterial, das aus der Gruppe bestehend aus
(a) menschlichen, tierischen und pflanzlichen Zellen,
(b) Fragmenten dieser Zellen, und
(c) synthetischen liposomhaltigen Zellwänden,
gewählt ist, mit einem entsprechenden flüssigen biologischen Medium in Kontakt gebracht werden, wobei das Zellmaterial, das einen durch die freien Radikale während der Lyse freisetzbaren Farbstoff enthält, zuvor mit einem chemischen oder physikalischen Agens in Kontakt gebracht wurde;
2) die Freisetzung der freien Radikale aus dem Erzeuger freier Radikale; und
3) die Lyse des Zellmaterials durch optische Dichtemessung, bezogen auf eine das Zellmaterial enthaltende Vergleichsprobe, die nicht mit dem chemischen oder physikalischen Agens in Kontakt gebracht wurde, beurteilt wird.

2. Verfahren nach Anspruch 1, **dadurch gekennzeichnet**, daß das Zellmaterial aus der Gruppe gewählt wird, die aus angefärbten Zellen menschlicher, tierischer oder pflanzlicher Herkunft besteht.

3. Verfahren nach Anspruch 2, **dadurch gekennzeichnet,** daß die angefärbten Zellen rote Blutkörperchen menschlicher oder tierischer Herkunft sind.

4. Verfahren nach Anspruch 1, **dadurch gekennzeichnet**, daß der Erzeuger freier Radikale aus der Gruppe gewählt wird, die aus Produkten bestehen, die freie Radikale mit einer Kinetik in der Größenordnung 0 oder 1 freisetzen.

5. Verfahren nach einem der Ansprüche 1 und 4**, dadurch gekennzeichnet,** daß der Erzeuger freier Radikale 2,2'-Azo-bis-(2-amidinopropan)dichlorhydrat ist.

6. Verfahren nach Anspruch 1, **dadurch gekennzeichnet**, daß 50 bis 200 mM des Erzeugers freier Radikale mit einem Zellmaterial in einer Konzentration von 10 bis 20 % p/v in einem wäßrigen biologischen Medium in Kontakt gebracht wird.

7. Verfahren nach einem der Ansprüche 1 und 6, **dadurch gekennzeichnet**, daß die freien Radikale durch Inkubieren bei 37 °C erzeugt werden.

## Claims

1. A method for evaluating the antioxidizing activities of a chemical or physical agent to be tested by a cell material having a known antioxidant activity, said method , which comprises using free oxidant radicals as a means of inducing cell lysis, being characterized in that
1°) a free radical generator is brought into contact, in an appropriate liquid biological medium, with a cell material selected from the group consisting of
(a) human, animal and plant cells,
(b) fragments of said cells, and
(c) liposomes containing synthetic walls,
said cell material, which contains a releasable pigment during the lysis by free radicals;
2°) the release of free radicals from said free radical generator is induced; and
3°) the lysis of the cell material by the free radicals is evaluated measuring the optic density by comparison with a control containing said cell material which has not been contaminated with the said chemical or physical agent.

2. A method according to claim 1, characterized in that the cell material is selected from the group consisting of pigmented cells of human, animal or vegetable origin.

3. A method according to claim 2, characterized in that said pigmented cells are erythrocytes of human or animal origin.

4. A method according to claim 1, characterized in that said free radical generator is selected from the group consisting of products which release free radicals according to a 0 or 1st order kinetics.

5. A method according to any one of claims 1 to 4, characterized in that said free radical generator is 2,2'-azo-bis(2-amidinopropane) dihydrochloride.

6. A method according to claim 1, characterized in that 50 to 200 mM of free radical generator are brought into contact with a cell material at a concentration of 10 to 20% w/v in an aqueous biological medium.

7. A method according to any one of claims 1 to 6, characterized in that the free radicals are generated by incubation at 37°C.
